# EUROPEAN PATENT SPECIFICATION

(11) **EP 3 371 291 B1**
(45) Date of publication and mention of the grant of the patent: **19.02.2020**
(21) Application number: 16862920.2
(22) Date of filing: 03.11.2016
(51) Int. Cl.: C11B 9/00, C07D 333/22, A23L 27/20, A61K 8/49, A61Q 11/00

(54) **FORMYLTHIOPHENES AND THEIR USE IN FLAVOR AND FRAGRANCE COMPOSITIONS**
FORMYLTHIOPHENE UND DEREN VERWENDUNG IN GESCHMACKS- UND DUFTSTOFFZUSAMMENSETZUNGEN
FORMYLTHIOPHÈNES ET LEUR UTILISATION DANS DES COMPOSITIONS D'ARÔME ET DE PARFUM

(30) Priority: 06.11.2015 US 201562251900 P
(43) Date of publication of application: 12.09.2018
(73) Proprietor: International Flavors & Fragrances Inc., New York, NY 10019 (US)
(72) Inventor: CANNON, Robert J., Fair Haven, New Jersey 07704 (US); JANCZUK, Adam Jan, Old Bridge, New Jersey 08857 (US); AGYEMANG, David O., Jackson, New Jersey 08527 (US); CURTO, Nicole L., Little Falls, New Jersey 07424 (US); CHEN, Zhen, Aberdeen, New Jersey 07747 (US); CAI, Tingwei, Holmdel, New Jersey 07733 (US); VUICH, Cynthia M., Belle Mead, New Jersey 08502 (US); NORDMAN, II, Kurt E., Randolph, New Jersey 07869 (US); DE ABREU, Ingo F.F., Princeton, New Jersey 08540 (US)
(74) Representative: Barker Brettell LLP
(86) International application number: PCT/US2016/060226
(87) International publication number: WO 2017/079368

(56) References cited:
- EP-A2- 1 692 952
- WO-A1-03/053953
- JP-A- S6 157 511
- JP-A- 2001 002 675
- US-A- 5 368 876
- US-A1- 2010 137 611
- US-A1- 2012 007 026
- HO C T ET AL: "Contribution of lipids to the formation of heterocyclic compounds [during non-enzymic browning] in model systems. TIOL- In 'Thermal generation of aromas' [see FSTA (1990) 22 11A21].", ACS SYMPOSIUM SERIES 1989 DEP. OF FOOD SCI., NEW JERSEY AGRIC. EXP. STA., COOK COLL., RUTGERS, STATE UNIV. OF NEW JERSEY, NEW BRUNSWICK, NJ 08903, USA, vol. 409, 1989, pages 105-113, XP002789929,
- R. ALAN AITKEN ET AL: "Unexpected Pyrolytic Behaviour of Substituted Benzo[c]thiopyran and Thieno[2,3-c]thiopyran S,S-dioxides", AUSTRALIAN JOURNAL OF CHEMISTRY: AN INTERNATIONAL JOURNAL FOR CHEMICAL SCIENCE, vol. 67, no. 8-9, 1 January 2014 (2014-01-01), pages 1288-1295, XP055382596, AU ISSN: 0004-9425, DOI: 10.1071/CH14155
- MICHAEL R DETTY AND DAVID S HAYS: "Studies towards Alkylthiophene-2-carboxaldehydes. Reduction of 3-Alkenylthiophenes with Triethylsilane/Trifluoroacetic Acid. Regioselectivity in Formylation Reactions of 3-Alkylthiophenes", HETEROCYCLES, JAPAN INSTITUTE OF HETEROCYCLIC CHEMISTRY, JP, vol. 40, no. 2, 1 January 1995 (1995-01-01), pages 925-938, XP009511993, ISSN: 0385-5414, DOI: 10.3987/COM-94-S93
- AITKEN, R. ALAN ET AL.: 'Unexpected Pyrolytic Behaviour of Substituted Benzo[c]thiopyran and Thieno[2,3-c]thiopyran S,S-dioxides' AUSTRALIAN JOURNAL OF CHEMISTRY vol. 67, no. 8-9, 30 May 2014, pages 1288 - 1295, XP055382596
- SHU, NA ET AL.: 'Identification of odour-active compounds in dried and roasted nori (Porphyra yezoensis) using a simplified gas chromatography-SNIF technique' FLAVOUR AND FRAGRANCE JOURNAL vol. 27, no. 2, 17 February 2012, pages 157 - 164, XP055382599
- KISS, MARIA ET AL.: 'Comparison of the fragrance constituents of Tuber aestivum and Tuber brumale gathered in Hungary' JOURNAL OF APPLIED BOTANY AND FOOD QUALITY vol. 84, no. 1, 2011, pages 102 - 110, XP055382602

## Description

### Field of the Invention

The present invention relates to new chemical entities and their use as flavor and fragrance materials.

### Background of the Invention

There is an ongoing need in the flavor industry for new flavor and aroma compounds that enhance or provide new flavors such as citrusy, citral-like, fresh and green sensation for food preparations. There is a similar ongoing need for fragrance chemicals to give perfumers and other persons the ability to create new fragrances for perfumes, colognes and personal care products. Those with skill in the art appreciate how differences in the chemical structures of the molecules can result in significant differences in the odor, notes and characteristics. For example, small structural differences between close analogs would result in molecules with distinctive flavor properties. These distinctive properties can be highly valuable as they provide unique and distinguished characters to flavor compositions. However, many of these distinctive properties can also be undesirable and, thus, would render molecules not suited for flavor use.

Flavouring and perfume compositions comprising thiophene compounds are disclosed in US Pat. No. 5,368,876.

### Summary of the Invention

The present invention provides novel chemicals, and their use to enhance the flavor of foodstuff, chewing gums, dental and oral hygiene products, medicinal products and the like. In addition, the present invention is also directed to the use of these novel chemicals to enhance the fragrance of perfumes, toilet waters, colognes, personal products and the like.

More specifically, the present invention provides a method of improving, enhancing or modifying a composition through the addition of an olfactory acceptable amount of a compound of Formula A: 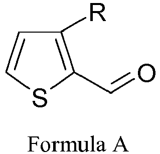
wherein R represents a C₄-C₈ straight or branched alkyl or alkenyl group; and
wherein the composition is a flavor or a fragrance composition.

In another aspect, the present invention provides a product comprising an olfactory acceptable amount of a compound of Formula A: 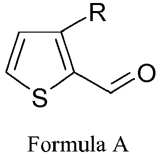 wherein R represents a C₄-C₈ straight or branched alkyl or alkenyl group; and
wherein the product is a cleaning product selected from the group consisting of a detergent, a dishwashing material, a scrubbing composition, a glass cleaner, a metal cleaner, a countertop cleaner, a floor cleaner, a carpet cleaner, a toilet cleaner and a bleach additive.

In another aspect, the present invention provides a compound selected from the group consisting of 3-(3-methyl-but-2-enyl)-2-formylthiophene and 3-(4-methyl-pent-3-enyl)-2-formylthiophene.

These and other embodiments of the present invention will be apparent by reading the following specification.

### Detailed Description of the Invention

The compounds of the present invention can be prepared, for example, according to the following reaction schemes, the details of which are specified in the Examples. Materials were purchased from Sigma-Aldrich Chemical Company unless noted otherwise.

The 3-substituted formylthiophene compounds of the present invention can be prepared, for example, according to the following procedures: 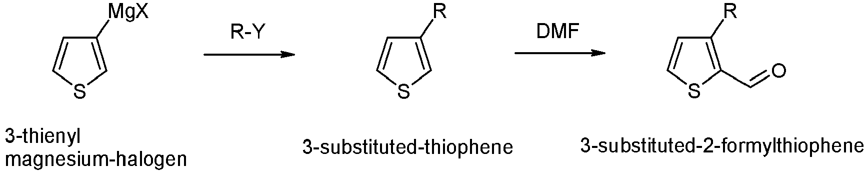
wherein X and Y can be the same or different and represent a halogen atom;
R is defined as above; and
DMF represents dimethylformamide.

Those with skill in the art will recognize that some of the compounds of the present invention contain chiral centers, thereby providing a number of isomers of the claimed compounds. It is intended herein that the compounds of the present invention include isomeric mixtures as well as individual isomers that may be separated using techniques known to those having skill in the art. In certain embodiments, positional isomers may also be included as the compounds of the present invention. Suitable techniques include chromatography such as high performance liquid chromatography, referred to as HPLC, particularly silica gel chromatograph, and gas chromatography trapping known as GC trapping. Yet, commercial versions of such products are mostly offered as mixtures.

The compounds of the present invention are found to have unexpected strong and long-lasting organoleptic properties such as, in particular, citrusy, citral-like, fresh and green characters, which are shown to be advantageous for their use in augmenting or imparting taste enhancement or somatosensory effect to foodstuffs, chewing gums, dental and oral hygiene products and medicinal products by providing flavor enhancement and a preferred overall flavor profile. The present invention further relates to a process of augmenting or imparting taste or somatosensory effect to foodstuffs, chewing gums, dental and oral hygiene products and medicinal products by adding the compounds of the present invention.

When the compounds of the present invention are used in a flavoring composition, they can be combined with conventional flavoring materials or adjuvants, which are well known in the art and have been extensively described in the past. Conventional flavoring materials include saturated fatty acids, unsaturated fatty acids, amino acids; alcohols including primary and secondary alcohols; esters; carbonyl compounds including ketones; aldehydes; lactones; cyclic organic materials including benzene derivatives, acyclic compounds, heterocyclies such as furans, pyridines, pyrazines and the like; sulfur-containing compounds including thiols, sulfides, disulfides and the like; proteins; lipids; carbohydrates; so-called flavor potentiators such as monosodium glutamate; magnesium glutamate, calcium glutamate, guanylates and inosinates; natural flavoring materials such as hydrolyzates, cocoa, vanilla and caramel; essential oils and extracts such as anise oil, clove oil and the like; and artificial flavoring materials such as vanillin, ethyl vanillin and the like. Requirements for adjuvants include: (1) that they be non-reactive with the 3-substituted formylthiophenes of the present invention; (2) that they be organoleptically compatible with the 3-substituted formylthiophenes of the present invention, whereby the flavor of the ultimate consumable product to which the 3-substituted formylthiophenes are added is not detrimentally affected by the use of the adjuvants; and (3) that they be ingestible acceptable and thus nontoxic or otherwise non-deleterious. In addition, other flavor materials, vehicles, stabilizers, thickeners, surface active agents, conditioners and flavor intensifiers can also be included.

The use of the compounds of the present invention is further applicable in current perfumery products, including the preparation of perfumes and colognes, the perfuming of personal care products such as soaps, shower gels, and hair care products, fabric care products, air fresheners, and cosmetic preparations. The present invention can also be used to perfume cleaning agents, such as, but not limited to detergents, dishwashing materials, scrubbing compositions, window cleaners and the like.

In these preparations, the compounds of the present invention can be used alone or in combination with other perfuming compositions, solvents, adjuvants and the like. The nature and variety of the other ingredients that can also be employed are known to those with skill in the art. Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in US Pat. No. 4,534,891. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

The compounds of the present invention can be used in combination with a complementary fragrance compound. The term "complementary fragrance compound" as used herein is defined as a fragrance compound selected from the group consisting of 2-[(4-methylphenyl)methylene]-heptanal (Acalea), iso-amyl oxyacetic acid allylester (Allyl Amyl Glycolate), (3,3-dimethylcyclohexyl)ethyl ethyl propane-1,3-dioate (Applelide), octahydro-4,7-methano-1H-indene-5-acetaldehyde; (E/Z)-1-ethoxy-1-decene (Arctical), 2-ethyl-4-(2,2,3-trimethyl-3-cyclo-penten-1-yl)-2-buten-1-ol (Bacdanol), 2-methyl-3-[(1,7,7-trimethylbicyclo[2.2.1]hept-2-yl)oxy]exo-1-propanol (Bornafix), 1,2,3,5,6,7-hexahydro-1,1,2,3,3-pentamethyl-4H-inden-4-one (Cashmeran), trimethylcyclopentenylmethyloxabicyclooctane (Cassiffix), 1,1-dimethoxy-3,7-dimethyl-2,6-octadiene (Citral DMA), 3,7-dimethyl-6-octen-1-ol (Citronellol), 3A,4,5,6,7,7A-hexahydro-4,7-methano-1*H*-inden-5/6-yl acetate (Cyclacet), 3A,4,5,6,7,7A-hexahydro-4,7-methano-1*H*-inden-5/6-yl propinoate (Cyclaprop), 3A,4,5,6,7,7A-hexahydro-4,7-methano-1G-inden-5/6-yl butyrate (Cyclobutanate), 1-(2,6,6-trimethyl-3-cyclohexen-1-yl)-2-buten-1-one (Delta Damascone), (1S)-1-[(1R)-3,3-dimethylcyclohexyl]ethyl ethyl malonate, 4-ethyloctanal, 3-(4-ethylphenyl)-2,2-dimethyl propanenitrile (Fleuranil), 3-(O/P-ethylphenyl) 2,2-dimethyl propionaldehyde (Floralozone), tetrahydro-4-methyl-2-(2-methylpropyl)-2H-pyran-4-ol (Floriffol), 1,3,4,6,7,8-hexahydro-4,6,6,7,8,8-hexamethylcyclopenta-gamma-2-benzopyran (Galaxolide), 1-(5,5-dimethyl-1-cyclohexen-1-yl)pent-4-en-1-one (Galbascone), E/Z-3,7-dimethyl-2,6-octadien-1-yl acetate (Geranyl Acetate), α-methyl-1,3-benzodioxole-5-propanal (Helional), 1-(2,6,6-trimethyl-2-cyclohexen-1-yl)-1,6-heptadien-3-one (Hexalon), (Z)-3-hexenyl-2-hydroxybenzoate (Hexenyl Salicylate, CIS-3), 4-(2,6,6-trimethyl-2-cyclohexen-1-yl)-3-buten-2-one (Ionone α), 1-(1,2,3,4,5,6,7,8-octahydro-2,3,8,8-tetramethyl-2-naphthalenyl)-ethan-1-one (Iso E Super), methyl 3-methylcyclohexanecarboxylate, methyl 3-oxo-2-pentylcyclopentaneacetate (Kharismal), 2,2,4-trimethyl-4-phenyl-butanenitrile (Khusinil), 3,4,5,6,6-pentamethylhept-3-en-2-one (Koavone), 3/4-(4-hydroxy-4-methyl-pentyl) cyclohexene-1-carboxaldehyde (Lyral), 3-methyl-4-(2,6,6-trimethyl-2-cyclohexen-1-yl)-3-buten-2-one (Methyl Ionone γ), 1-(2,6,6-trimethyl-2-cyclohexen-1-yl) pent-1-en-3-one (Methyl Ionone α Extra, Methyl Ionone N), [(Z)-hex-3-enyl] cyclopropanecarboxylate, 3-methyl-4-phenylbutan-2-ol (Muguesia), cyclopentadec-4-en-1-one (Musk Z4), 3,3,4,5,5-pentamethyl-11,13-dioxatricyclo[7.4.0.0<2,6>]tridec-2(6)-ene (Nebulone), 3,7-dimethyl-2,6-octadien-1-yl acetate (Neryl Acetate), 3,7-dimethyl-1,3,6-octatriene (Ocimene), dec-6 or 7 or 8-enal, 2,2,6,6,7,8,8-heptamethyl-4,5,6,7,8,8B-hexahydro-3AH-indeno[4,5-D][1,3]dioxole, cis-1-(1,1-dimethylpropyl)-4-ethoxy cyclohexane, ortho-tolylethanol (Peomosa), 3-methyl-5-phenylpentanol (Phenoxanol), 1-methyl-4-(4-methyl-3-pentenyl) cyclohex-3-ene-1-carboxaldehyde (Precyclemone B), 4-methyl-8-methylene-2-adamantanol (Prismantol), 2-ethyl-4-(2,2,3-trimethyl-3-cyclopenten-1-yl)-2-buten-1-ol (Sanjinol), 2-methyl-4-(2,2,3-trimethyl-3-cyclopenten-1-yl)-2-buten-1-ol (Santaliff), 7,7,8,9,9-pentamethyl-6,7,8,9-tetrahydro-5H-cyclopenta[H]quinazoline, 3-[cis-4-(2-methylpropyl)cyclohexyl]propanal, 4-(heptyloxy)-3-methylbutanal, Terpineol, 2,4-dimethyl-3-cyclohexene-1-carboxaldehyde (Triplal), decahydro-2,6,6,7,8,8-hexamethyl-2H-indeno[4,5-B]furan (Trisamber), ethyl bicyclo[2.2.1]hept-5-ene-2-carboxylate, 3,4,5-trimethyloctahydro-1H-4,7-methanoinden-5-ol, 2-tert-butylcyclohexyl acetate (Verdox), (3E)-4-methyldec-3-en-5-one, 4-tert-butylcyclohexyL acetate (Vertenex), acetyl cedrene (Vertofix), 3,6/4,6-dimethylcyclohex-3-ene-1-carboxaldehyde (Vertoliff) and (3Z)-1-[(2-methyl-2-propenyl)oxy]-3-hexene (Vivaldie).

The term "foodstuff" as used herein includes both solid and liquid ingestible materials for man or animals, which materials usually do, but need not, have nutritional value. Thus, foodstuffs include meats, gravies, soups, convenience foods, malt, alcoholic and other beverages, milk and dairy products, seafood, including fish, crustaceans, mollusks and the like, candies, vegetables, cereals, soft drinks, snacks, dog and cat foods, other veterinary products and the like.

The terms "flavor composition" and "flavor formulation" mean the same and refer to a consumer composition that produces a pleasant or desired flavor. The flavor composition contains a compound or a mixture of compounds. The flavor composition in the method of the present invention is a consumer composition comprising a compound of the present invention.

The term "flavor product" means a consumer product containing a compound of the present invention and further a foodstuff, a chewing gum, a dental product, an oral hygiene product or a medicinal product.

The terms "fragrance composition", "fragrance formulation" and "perfume composition" mean the same and refer to a consumer composition that is a mixture of compounds including, for example, alcohols, aldehydes, ketones, esters, ethers, lactones, nitriles, natural oils, synthetic oils, and mercaptans, which are admixed so that the combined odors of the individual components produce a pleasant or desired fragrance. The fragrance composition of the present invention is a consumer composition comprising a compound of the present invention. The fragrance composition in the method of the present invention comprises a compound of the present invention and further a complementary fragrance compound as defined above.

The term "fragrance product" means a consumer product containing a fragrance ingredient that adds fragrance or masks malodor. Fragrance products may include, for example, perfumes, colognes, toilet water, bar soaps, liquid soaps, shower gels, foam baths, cosmetic products, personal care products including skin care products such as creams, lotions and shaving products, hair care products for shampooing, rinsing, conditioning, bleaching, coloring, dyeing and styling, deodorants and antiperspirants, feminine care products such as tampons and feminine napkins, baby care products such as diapers, bibs and wipes, family care products such as bath tissues, facial tissues, paper handkerchiefs or paper towels, fabric care products such as fabric softeners and fresheners, air care products such as air fresheners and fragrance delivery systems, cosmetic preparations, cleaning products and disinfectants such as detergents, dishwashing materials, scrubbing compositions, glass and metal cleaners such as window cleaners, countertop cleaners, floor and carpet cleaners, toilet cleaners and bleach additives, washing agents such as all-purpose, heavy duty, and hand washing or fine fabric washing agents including laundry detergents and rinse additives, dental and oral hygiene products such as toothpastes, tooth gels, dental flosses, denture cleansers, denture adhesives, dentifrices, tooth whitening and mouthwashes, health care and nutritional products and food products such as snack and beverage products. The fragrance product of the present invention is a cleaning product that contains a compound of the present invention. The fragrance product of the present invention contains a compound of the present invention and further a complementary fragrance compound as defined above.

The term "improving" is understood to mean raising a flavor or fragrance composition to a more desirable character. The term "enhancing" is understood to mean making the flavor or fragrance composition greater in effectiveness or providing the flavor or fragrance composition with an improved character. The term "modifying" is understood to mean providing the flavor or fragrance composition with a change in character.

The term "olfactory acceptable amount" is understood to mean the amount of a compound in a flavor or fragrance formulation, wherein the compound will contribute its individual olfactory characteristics. However, the olfactory effect of the flavor or fragrance formulation will be the sum of effect of each of the flavor or fragrance ingredients. Thus, the compound of the present invention can be used to improve or enhance the aroma characteristics of the flavor or fragrance formulation, or by modifying the olfactory reaction contributed by other ingredients in the formulation. The olfactory acceptable amount may vary depending on many factors including other ingredients, their relative amounts and the olfactory effect that is desired.

Generally, the olfactory acceptable amount of the 3-substituted formylthiophenes employed in a flavor composition is greater than about 0.1 parts per billion by weight, preferably from about 1 part per billion to about 500 parts per million by weight, more preferably from about 10 parts per billion to about 100 parts per million by weight, even more preferably from about 100 parts per billion to about 50 parts per million by weight. The olfactory acceptable amount of the 3-substituted formylthiophenes employed in a fragrance composition varies from about 0.005 to about 70 weight percent, preferably from 0.005 to about 50 weight percent, more preferably from about 0.5 to about 25 weight percent, and even more preferably from about 1 to about 10 weight percent. Those with skill in the art will be able to employ the desired amount to provide desired flavor or fragrance effect and intensity. In addition to the compounds of the present invention, other materials can also be used in conjunction with the flavor or fragrance composition to encapsulate and/or deliver the flavor or fragrance. Some well-known materials are, for example, but not limited to, polymers, oligomers, other non-polymers such as surfactants, emulsifiers, lipids including fats, waxes and phospholipids, organic oils, mineral oils, petrolatum, natural oils, perfume fixatives, fibers, starches, sugars and solid surface materials such as zeolite and silica.

The following are provided as specific embodiments of the present invention. Other modifications of this invention will be readily apparent to those skilled in the art. Such modifications are understood to be within the scope of this invention. As used herein all percentages are weight percent unless otherwise noted, ppb is understood to stand for parts per billion, ppm is understood to stand for parts per million, L is understood to be liter, mL is understood to be milliliter, Kg is understood to be kilogram, g is understood to be gram, mol is understood to be mole, mmol is understood to be millimole and M is understood to be molar. IFF as used in the examples is understood to mean International Flavors & Fragrances Inc., New York, NY, USA.

### EXAMPLE I

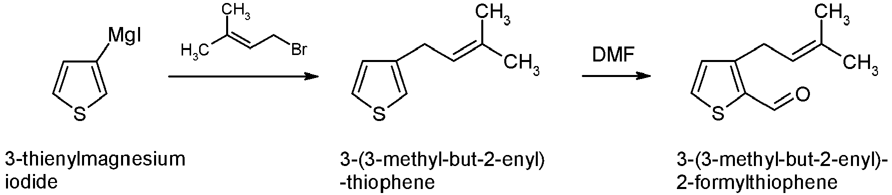

**Preparation of 3-(3-Methyl-but-2-enyl)-2-formylthiophene (Structure B1):** A 2 L, four-necked flask was charged with 1-bromo-3-methylbut-2-ene (10 g, 67 mmol) in ethyl ether ((C₂H₅)₂O) (75 mL) to yield a colorless solution. [1,2-Bis(diphenylphosphino)ethane]dichloronickel(II) (dppeNiCl₂) (0.5 g, 1 mmol) was added and the mixture was allowed to stir at 0 °C. 3-Thienylmagnesium iodide (224 mL, 67 mmol) was added dropwise at 0 °C. The reaction mixture was allowed to warm to room temperature and stirred for 8 hours. After the reaction was complete, ice was added along with an aqueous hydrochloric acid (HCl) solution (10%). The reaction mixture was extracted with ether. The organic layer was separated, washed with brine, dried over magnesium sulfate (MgSO₄) and concentrated. Purification of the crude with Kugelrohr distillation provided 3-(3-methyl-but-2-enyl)-thiophene (22 g). A 500 mL, four-neck flask was charged with dimethylformamide ((CH₃)₂NC(O)H) (DMF) (9.6 g, 131 mmol) and the temperature was allowed to cool to 0 °C with stirring. Phosphorus oxychloride (POCl₃) (2.8 g, 18 mmol) was added dropwise while the temperature was kept blow 10 °C using an ice bath. The mixture was stirred for additional 15 minutes. 3-(3-Methyl-but-2-enyl)-thiophene (2.5 g, 16 mmol) was then added dropwise at 10 °C. The ice bath was removed and the reaction mixture was heated to 70 °C and stirred for 6 hours. After the reaction was complete, ice was added along with an aqueous sodium hydroxide (NaOH) solution (5%). The reaction mixture was extracted with methyl tert-butyl ether ((CH₃)₃COCH₃) (300 mL). The organic layer was separated, washed with water and brine, dried over MgSO₄ and concentrated. The resulting crude was purified by vacuum distillation and Biotage (hexane:ethyl acetate = 4:1) to afford 3-(3-methyl-but-2-enyl)-2-formylthiophene (0.5 g).
¹H NMR (CDCl₃, 400-MHz): 10.07 ppm (s, 1H), 7.64 ppm (d, J=5.0 Hz, 1H), 7.01 ppm (d, J=5.0 Hz, 1H), 5.25-5.39 ppm (m, 1H), 3.68 ppm (d, J=7.2 Hz, 2H), 1.67-1.82 ppm (m, 6H)

### EXAMPLE II

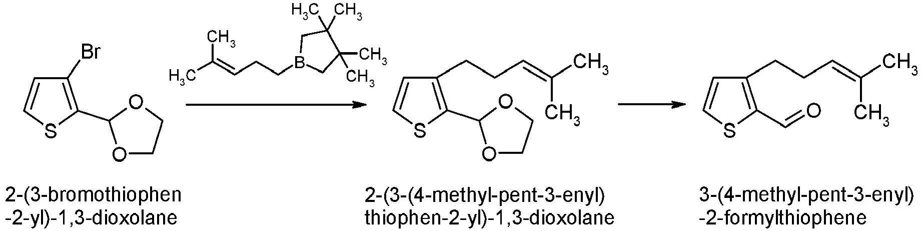

**Preparation of 3-(4-Methyl-pent-3-enyl)-2-formylthiophene (Structure B2):** To a mixture of 2-(3-bromothiophen-2-yl)-1,3-dioxolane (C₇H₇BrO₂S) (4.3 g, 18.29 mmol), 4-methyl-3-pentenylboronic acid pinacol ester (C₁₂H₂₃BO₂) (5 g, 23.78 mmol), and potassium carbonate (K₂CO₃) (7.58 g, 54.9 mmol) in toluene (65 mL) was added water (9.5 mL), 2-dicyclohexylphosphino-2',6'-diisopropoxybiphenyl (RuPhos) (0.34 g, 0.73 mmol) and palladium (II) acetate (Pd(O₂CCH₃)₂) (82 mg, 0.37 mmol) at room temperature under nitrogen. The reaction mixture was heated to 120 °C and stirred for 6 hours under nitrogen. After the reaction was cooled to room temperature, the reaction mixture was filtered, washed with ethyl acetate (CH₃CO₂C₂H₅) and water and then extracted with ethyl acetate. The organic layer was separated, washed with brine and dried over MgSO₄. The crude product was purified by chromatography on silica gel eluting with ethyl acetate/hexane to afford 2-(3-(4-methyl-pent-3-enyl)thiophen-2-yl)-1,3-dioxolane, which was re-dissolved in toluene (25 mL) and added with concentrated hydrochloric acid (HCl) (2 mL). The reaction mixture was stirred at room temperature for 1 hour, quenched with sodium bicarbonate (NaHCO₃) solution and extracted with ether. The organic layer was separated, washed subsequently with water and brine and dried over MgSO₄. The solvent was removed under reduced pressure and the resulting crude was purified by distillation to afford 3-(4-methyl-pent-3-enyl)-2-formylthiophene (1 g) as a yellow liquid.
¹H NMR (CDCl₃, 400-MHz): δ 9.97 (d, *J*=1.0 Hz, 1H), 7.63 (d, *J*=4.9 Hz, 1H), 7.01 (d, *J*=4.9 Hz, 1H), 5.13 (t, *J*=7.3 Hz, 1H), 2.98 (t, *J*=7.4 Hz, 2H), 2.33 (dt, *J*=7.4, 7.3 Hz, 2H), 1.67 (s, 3 H), 1.45 (s, 3H)

### EXAMPLE III

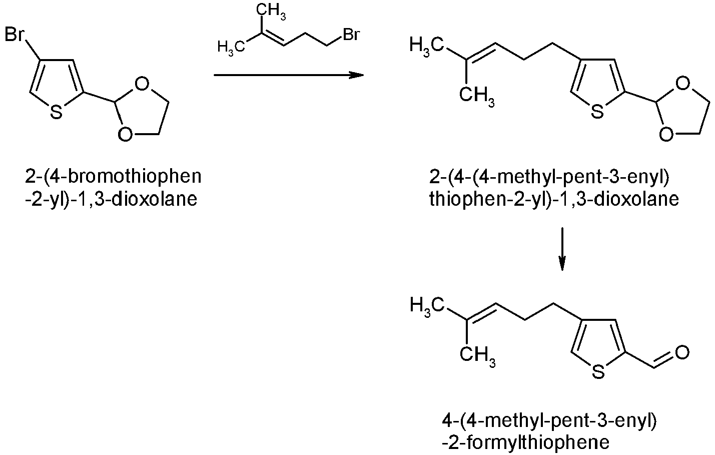

**Preparation of 4-(4-Methyl-pent-3-enyl)-2-formylthiophene (Structure B3):** To a mixture of nickel(II) iodide (NiI₂) (0.17 g, 0.55 mmol), water (32 mg, 1.75 mmol), sodium iodide (Nal) (0.19 g, 1.25 mmol), pyridine (C₅H₅N) (0.79 g, 10 mmol), 5-bromo-2-methylpent-2-ene (Br(CH₂)₂CHC(CH₃)₂) (0.82 g, 5 mmol), 1,10-phenanthroline (C₁₂H₈N₂) (0.09 g, 0.5 mmol) and 2-(4-bromothiophen-2-yl)-1,3-dioxolane (1.18 g, 5 mmol) in 1,3-dimethyltetrahydropyrimidin-2(1H)-one (DMPU) (20 g) was added zinc dust (0.65 g, 10 mmol) at room temperature. The reaction mixture was stirred at room temperature for 5 minutes, heated to 70 °C and stirred for another 20 hours. After the reaction was cooled to room temperature, the reaction mixture was poured into water (100 mL) and extracted with ether (50 mL). The organic layer was separated, washed with NaHCO₃ solution and dried over MgSO₄. The crude product was purified by chromatography on silica gel eluting with ethyl acetate/hexane to afford 2-(4-(4-methyl-pent-3-enyl)thiophen-2-yl)-1,3-dioxolane (0.5 g) as a clear liquid. The final product 4-(4-methyl-pent-3-enyl)-2-formylthiophene (0.3 g) was subsequently prepared similarly according to EXAMPLE II from 2-(4-(4-methyl-pent-3-enyl)thiophen-2-yl)-1,3-dioxolane.
¹H NMR (CDCl₃, 400-MHz): 9.89 (d, J= 1.2 Hz, 1H), 7.62 (d, J= 1.4 Hz, 1H), 7.39 (s, 1H), 5.15 (t, J= 7.1 Hz, 1H), 2.69 (t, J= 7.6 Hz, 2H), 2.33 (dt, J= 7.5, 7.3 Hz, 2H), 1.71 (s, 3 H), 1.57 (s, 3H)

### EXAMPLE IV

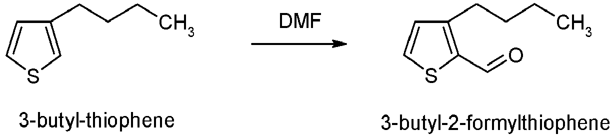

**Preparation of 3-Butyl-2-formylthiophene (Structure C1):** A 1 L, three-neck flask was charged with DMF (208 g, 2.852 mol). Under nitrogen gas (N₂), POCl₃ (60 g, 392 mmol) was added slowly while the temperature was kept below 10 °C using an ice bath. After the addition was complete, the ice bath was removed and the reaction mixture was allowed to warm to room temperature and stirred for 10 minutes. 3-Butylthiophene (50 g, 357 mmol) was added. The reaction mixture was stirred at room temperature for 10 minutes and then heated to 70 °C and stirred for additional 4.5 hours. The resulting reaction mixture was added to a NaOH solution (5%, 400 mL) and extracted with methyl-tert-butyl ether (600 mL). The organic layer was separated, washed with brine and dried over MgSO₄. Further purification of the crude with distillation (98 °C /2.86 mm Hg) afforded 3-butyl-2-formylthiophene (45.6 g).
¹H NMR (CDCl₃, 400-MHz): 10.02-10.07 ppm (m, 1H), 7.64 ppm (d, J=5.0 Hz, 1H), 7.01 ppm (d, J=5.0 Hz, 1H), 2.97 ppm (t, J=7.9 Hz, 2H), 1.57-1.77 ppm (m, 2H), 1.28-1.47 ppm (m, 2H), 0.94 ppm (t, J=7.3 Hz, 3H)

### EXAMPLE V

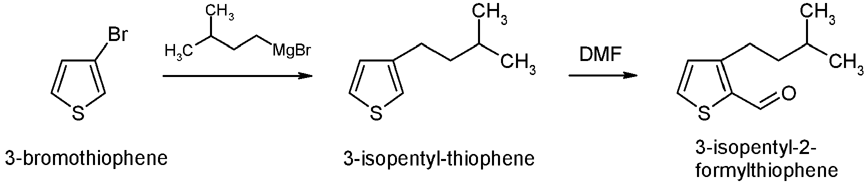

**Preparation of 3-Isopentyl-2-formylthiophene (Structure C2):** A 500 mL, four-neck flask was charged with 3-bromothiophene (14 g, 83 mmol), dppeNiCl₂ (0.9 g, 1.7 mmol) and tetrahydrofuran (THF) (CH₂)₄O (100 mL). Isopentylmagnesium bromide (18 g, 100 mmol) was diluted in THF (100 mL) and added dropwise into the mixture at room temperature. The resulting mixture was then stirred for 2 hours with refluxing. After the reaction was complete, a NaOH solution (10%, 200 mL) was added followed by extraction with methyl tert-butyl ether (300 mL). The organic layer was separated, washed twice with brine and dried over MgSO₄. Crude 3-isopentyl-thiophene was obtained and purified with Kugelrohr distillation (80 °C / 7 mmHg). A 500 mL, three-neck flask was charged with DMF (19 g, 259 mmol). Under N₂, POCl₃ (5 g, 36 mmol) was slowly added while the temperature was kept below 10 °C using an ice bath. After the addition was complete, the ice bath was removed and the reaction mixture was allowed to stir at room temperature for 10 minutes. 3-Isopentyl-thiophene (5 g, 32 mmol) was then added dropwise and the reaction mixture was heated to 65 °C and stirred for 8 hours. The resulting mixture was added to a NaOH solution (5%, 20 mL) and extracted with ethyl acetate (100 mL). The organic layer was separated, washed with brine and dried over MgSO₄. Further purification with a short silica column (hexane:ethyl acetate = 10:1) afforded 3-isopentyl-2-formylthiophene (3.2 g).
¹H NMR (CDCl₃, 500-MHz): 10.01-10.09 ppm (m, 1H), 7.63 ppm (dd, J=5.0 Hz, 1H), 7.00 ppm (d, J=5.0 Hz, 1H), 2.92-3.01 ppm (m, 2H), 1.49-1.66 ppm (m, 3H), 0.95 ppm (d, J=6.3 Hz, 6H)

### EXAMPLE VI

Following formylthiophenes were similarly prepared.

### 3-Isobutyl-2-formylthiophene (Structure C3):

¹H NMR (CDCl₃, 500-MHz): 10.01 ppm (d, J=0.9 Hz, 1H), 7.63 ppm (dd, J=5.0, 0.9 Hz, 1H), 6.97 ppm (d, J=5.0 Hz, 1H), 2.83 ppm (d, J=6.9 Hz, 2H), 1.90-1.98 ppm (m, 1H), 0.94 ppm (d, J=6.6 Hz, 6H)

### 3-Pentyl-2-formylthiophene (Structure C4):

¹H NMR (CDCl₃, 400-MHz): 9.99-10.12 ppm (m, 1H), 7.63 ppm (d, J=5.0 Hz, 1H), 7.01 ppm (d, J=5.0 Hz, 1H), 2.96 ppm (t, J=7.7 Hz, 2H), 1.67 ppm (m, 2H), 1.24-1.41 ppm (m, 4H), 0.81-1.02 ppm (m, 3H)

### 3-Hexyl-2-formylthiophene (Structure C5):

¹H NMR (CDCl₃, 400-MHz): 10.04 ppm (d, J=0.9 Hz, 1H), 7.63 ppm (dd, J=4.9, 0.9 Hz, 1H), 7.01 ppm (d, J=5.0 Hz, 1H), 2.91-3.02 ppm (m, 2H), 1.57-1.74 ppm (m, 2H), 1.21-1.45 ppm (m, 6H), 0.88 ppm (t, J=6.9 Hz, 3H)

### 3-Heptyl-2-formylthiophene (Structure C6):

¹H NMR (CDCl₃, 400-MHz): 10.03 ppm (s, 1H), 7.62 ppm (d, J=4.5 Hz, 1H), 7.00 ppm (d, J=4.9 Hz, 1H), 2.95 ppm (t, J=7.7 Hz, 2H), 1.58-1.74 ppm (m, 2H), 1.22-1.38 ppm (m, 8H), 0.87 ppm (t, J=6.8 Hz, 3H)

### 3-Octyl-2-formylthiophene (Structure C7):

¹H NMR (CDCl₃, 400-MHz): 10.02 ppm (d, J=0.9 Hz, 1H), 7.61 ppm (dd, J=4.9, 0.9 Hz, 1H), 6.99 ppm (d, J=5.0 Hz, 1H), 2.89-3.02 ppm (m, 2H), 1.58-1.71 ppm (m, 2H), 1.20-1.37 ppm (m, 10H), 0.86 ppm (t, J=7.3 Hz, 3H)

### 2-Formylthiophene (Structure 1) and 3-Methyl-2-formylthiophene (Structure 2):

Purchased from Sigma-Aldrich Chemical Company.

### 3-Ethyl-2-formylthiophene (Structure 3):

¹H NMR (CDCl₃, 500-MHz): 10.03 ppm (d, J=0.9 Hz, 1H), 7.63 ppm (dd, J=5.0, 0.9 Hz, 1H), 7.02 ppm (d, J=5.0 Hz, 1H), 2.98 ppm (q, J=7.7 Hz, 2H), 1.29 ppm (t, J=7.6 Hz, 3H)

### 3-Propyl-2-formylthiophene (Structure 4):

¹H NMR (CDCl₃, 400-MHz): 10.04 ppm (s, 1H), 7.64 ppm (d, J=4.9 Hz, 1H), 7.01 ppm (d, J=4.9 Hz, 1H), 2.95 ppm (t, J=7.5 Hz, 2H), 1.65-1.78 ppm (m, 2H), 0.97 ppm (t, J=7.3 Hz, 3H)

### 3-Nonyl-2-formylthiophene (Structure 5):

¹H NMR (CDCl₃, 400-MHz): 10.05 ppm (d, J=0.8 Hz, 1H), 7.65 ppm (d, J=4.8 Hz, 1H), 7.02 ppm (d, J=4.9 Hz, 1H), 2.97 ppm (t, J=7.7 Hz, 2H), 1.61-1.76 ppm (m, 2H), 1.21-1.46 ppm (m, 12H), 0.89 ppm (t, J=6.7 Hz, 3H)

### 3-Decyl-2-formylthiophene (Structure 6):

¹H NMR (CDCl₃, 400-MHz): 10.02 ppm (d, J=0.9 Hz, 1H), 7.61 ppm (d, J=4.9 Hz, 1H), 6.99 ppm (d, J=4.9 Hz, 1H), 2.94 ppm (t, J=7.7 Hz, 2H), 1.55-1.74 ppm (m, 2H), 1.17-1.42 ppm (m, 14H), 0.86 ppm (t, J=6.8 Hz, 3H)

### 3-Propenyl-2-formylthiophene (Structure 7):

¹H NMR (CDCl₃, 500-MHz): 10.02 ppm (d, J=0.9 Hz, 1H), 7.64 ppm (dd, J=5.4, 0.9 Hz, 1H), 7.23 ppm (d, J=5.4 Hz, 1H), 5.87-6.06 ppm (m, 1H), 5.02-5.22 ppm (m, 2H), 3.72 ppm (dt, J=6.5, 1.3 Hz, 2H)

### 3-Prop-1-ynyl-2-formylthiophene (Structure 8):

¹H NMR (CDCl₃, 400-MHz): 9.98 ppm (d, J=1.4 Hz, 1H), 7.53 ppm (dd, J=5.0, 1.4 Hz, 1H), 7.00 ppm (d, J=5.0 Hz, 1H), 2.00 ppm (s, 3H)

### EXAMPLE VII

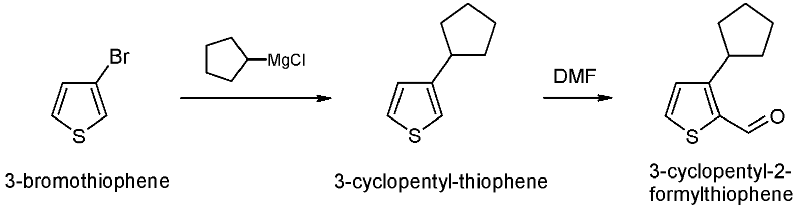

**Preparation of 3-Cyclopentyl-2-formylthiophene (Structure 9):** 3-Cyclopentyl-thiophene was first prepared with 3-bromothiophene and cyclopentylmagnesium chloride according to the procedures described above. 3-Cyclopentyl-2-formylthiophene was subsequently prepared with 3-cyclopentyl-thiophene and DMF.
¹H NMR (CDCl₃, 400-MHz): 9.99 ppm (s, 1H), 7.52 ppm (d, J=5.1 Hz, 1H), 6.97 ppm (d, J=5.1 Hz, 1H), 3.50-3.65 ppm (m, 1H), 1.93-2.08 ppm (m, 2H), 1.38-1.77 ppm (m, 6H)

### EXAMPLE VIII

Following formylthiophenes were similarly prepared.

### 3-Cyclopropyl-2-formylthiophene (Structure 10):

¹H NMR (CDCl₃, 400-MHz): 10.21 ppm (s, 1H), 7.60 ppm (d, J=5.0 Hz, 1H), 6.68 ppm (d, J=5.0 Hz, 1H), 2.45-2.56 ppm (m, 1H), 1.11-1.24 ppm (dd, J=8.4, 1.8 Hz, 2H), 0.83-0.92 ppm (m, 2H)

### 3-Cyclohexyl-2-formylthiophene (Structure 11):

¹H NMR (CDCl₃, 400-MHz): 10.10 ppm (s, 1H), 7.64 ppm (d, J=5.0 Hz, 1H), 7.10 ppm (d, J=5.0 Hz, 1H), 3.22-3.34 ppm (m, 1H), 1.71-2.06 ppm (m, 5H), 1.21-1.60 ppm (m, 5H)

### EXAMPLE IX

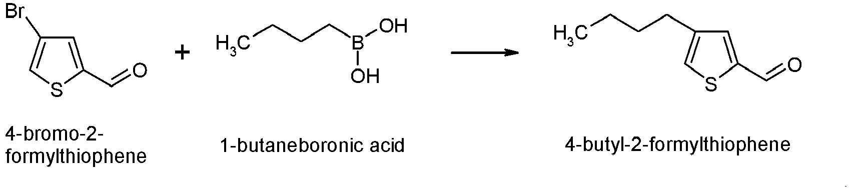

**Preparation of 4-butyl-2-formylthiophene (Structure 12):** A reactor was charged with a solution of toluene in water in a weight ratio of 9:1 (150 mL), 4-bromo-2-formylthiophene (6.3 g, 33 mmol), 1-butaneboronic acid (5 g, 49 mmol), palladium(II) acetate (Pd(OAc)₂) (0.1 g, 0.6 mmol), 2-dicyclohexylphosphino-2',6'-diisopropoxybiphenyl (RuPhos) (C₃₀H₄₃O₂P) (0.6 g, 1.3 mmol) and potassium carbonate anhydrous (K₂CO₃) (13.6 g, 98 mmol). The reactor was sealed and heated to 150 °C. The reaction was carried out for 6 hours. After the reaction was complete, ethyl acetate (100 mL) was added. The reaction mixture was washed twice with brine. The organic layer was separated, dried over MgSO₄ and concentrated. The resulting crude was purified with Kugelrohr distillation (140-150 °C / 1.1 mmHg) to afford 4-butyl-2-formylthiophene (6.4 g).
¹H NMR (CDCl₃, 500-MHz): 9.86 ppm (d, J=1.3 Hz, 1H), 7.60 ppm (d, J=1.6 Hz, 1H), 7.34-7.38 ppm (m, 1H), 2.64 ppm (t, J=7.7 Hz, 2H), 1.57-1.68 ppm (m, 2H), 1.28-1.48 ppm (m, 2H), 0.93 ppm (t, J=7.3 Hz, 3H)

### EXAMPLE X

Following formylthiophenes were similarly prepared.

### 4-Ethyl-2-formylthiophene (Structure 13):

¹H NMR (CDCl₃, 500-MHz): 9.85 ppm (d, J=0.9 Hz, 1H), 7.61 ppm (d, J=1.6 Hz, 1H), 7.30-7.41 ppm (m, 1H), 2.66 ppm (q, J=7.7 Hz, 2H), 1.25 ppm (t, J=7.6 Hz, 3H)

### 4-Propyl-2-formylthiophene (Structure 14):

¹H NMR (CDCl₃, 500-MHz): 9.87 ppm (d, J=1.3 Hz, 1H), 7.60 ppm (d, J=1.3 Hz, 1H), 7.36-7.38 ppm (m, 1H), 2.62 ppm (t, J=7.6 Hz, 2H), 1.61-1.70 ppm (m, 2H), 0.96 ppm (t, J=7.3 Hz, 3H)

### 4-Isobutyl-2-formylthiophene (Structure 15):

¹H NMR (CDCl₃, 500-MHz): 9.87 ppm (d, J=1.3 Hz, 1H), 7.57 ppm (d, J=1.6 Hz, 1H), 7.33-7.37 ppm (m, 1H), 2.51 ppm (d, J=7.3 Hz, 2H), 1.83-1.90 ppm (m, 1H), 0.91 ppm (d, J=6.6 Hz, 6H)

### 4-Pentyl-2-formylthiophene (Structure 16):

¹H NMR (CDCl₃, 400-MHz): 9.88 ppm (d, J=1.1 Hz, 1H), 7.62 ppm (d, J=1.3 Hz, 1H), 7.36-7.40 ppm (m, 1H), 2.65 ppm (t, J=7.7 Hz, 2H), 1.59-1.71 ppm (m, 2H), 1.25-1.41 ppm (m, 4H), 0.91 ppm (t, J=6.3 Hz, 4H)

### 4-Hexyl-2-formylthiophene (Structure 17):

¹H NMR (CDCl₃, 400-MHz): 9.89 ppm (s, 1H), 7.62 ppm (d, J=0.9 Hz, 1H), 7.39 ppm (s, 1H), 2.66 ppm (t, J=7.7 Hz, 2H), 1.59-1.78 ppm (m, 2H), 1.26-1.46 ppm (m, 6H), 0.91 ppm (t, J=6.9 Hz, 3H)

### EXAMPLE XI

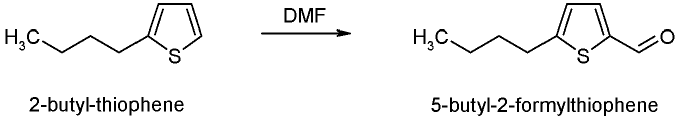

**Preparation of 5-Butyl-2-formylthiophene (Structure 18):** A round bottom flask was charged with DMF (21 g, 285 mmol). Under N₂, POCl₃ (6.0 g, 39 mmol) was slowly added while the temperature was kept below 10 °C using an ice bath. After the addition was complete, the ice bath was removed and the solution was stirred for 10 minutes at room temperature. 2-Butylthiophene (5 g, 36 mmol) was added dropwise and the reaction mixture was heated to 65 °C for 8 hours. The reaction mixture was then poured into a saturated sodium carbonate (Na₂CO₃) solution (20 mL) and extracted with ethyl acetate (100 mL). The resulting organic layer was separated, washed with brine and dried over MgSO₄. The crude was concentrated and purified using a short silica gel column to afford 5-butyl-2-formylthiophene (4 g).
¹H NMR (CDCl₃, 400-MHz): 9.60-9.82 ppm (m, 1H), 7.44-7.62 ppm (m, 1H), 6.70-6.93 ppm (m, 1H), 2.76 ppm (t, J=7.8 Hz, 2H), 1.57 ppm (m, 2H), 1.28 ppm (m, 2H), 0.83 ppm (t, J=7.4 Hz, 3H)

### EXAMPLE XII

Following formylthiophenes were similarly prepared.

### 5-Methyl-2-formylthiophene (Structure 19):

Purchased from Sigma-Aldrich Chemical Company.

### 5-Pentyl-2-formylthiophene (Structure 20):

¹H NMR (CDCl₃, 400-MHz): 9.70-10.01 ppm (m, 1H), 7.62 ppm (d, J=3.7 Hz, 1H), 6.91 ppm (d, J=3.7 Hz, 1H), 2.88 ppm (t, J=7.6 Hz, 2H), 1.58-1.91 ppm (m, 2H), 1.24-1.48 ppm (m, 4H), 0.91 ppm (t, J=7.0 Hz, 3H)

### 5-Hexyl-2-formylthiophene (Structure 21):

¹H NMR (CDCl₃, 500-MHz): 9.80 ppm (s, 1H), 7.59 ppm (d, J=3.7 Hz, 1H), 6.89 ppm (d, J=3.7 Hz, 1H), 2.85 ppm (t, J=7.3 Hz, 2H), 1.63-1.76 ppm (m, 2H), 1.25-1.43 ppm (m, 6H), 0.86 ppm (t, J=7.3 Hz, 3H)

### 2-Propyl-3-formylthiophene (Structure 22):

2-Propyl-3-formylthiophene was prepared according to the procedures described in Naf, et al. (Flavour & Fragrance Journal. 2000 15(5): 329-334).
¹H NMR (CDCl₃, 500-MHz): 10.03 ppm (s, 1H), 7.38 ppm (d, J=5.4 Hz, 1H), 7.08 ppm (d, J=5.4 Hz, 1H), 3.15 ppm (t, J=7.6 Hz, 2H), 1.72-1.81 ppm (m, 2H), 1.01 ppm (t, J=7.4 Hz, 3H)

### EXAMPLE XIII

The organoleptic properties of the above formylthiophenes were evaluated and are reported in the following:

| **Chemical Name** | **Organoleptic Profile** |
|---|---|
| 3-(3-Methyl-but-2-enyl)-2-formylthiophene (Structure B1) | Citrusy, citral-like, fresh, green and sweet |
| 3-(4-Methyl-pent-3-enyl)-2-formylthiophene (Structure B2) | Citral-like, citronellal-like, floral and sweet |
| 4-(4-Methyl-pent-3-enyl)-2-formylthiophene (Structure B3) | Citral-like, solventy, fatty, oxidized, mouth coating and drying |
| 3-Butyl-2-formylthiophene (Structure C1) | Citrusy, citral-like, peely, green, sweet and heavy/fatty mouthfeel |
| 3-Isopentyl-2-formylthiophene (Structure C2) | Strong, citrusy, citral-like, green, floral, fatty mouthfeel and spicy |
| 3-Isobutyl-2-formylthiophene (Structure C3) | Citrusy, citral-like, fruity, nutty and fatty mouthfeel, woody and spicy |
| 3-Pentyl-2-formylthiophene (Structure C4) | Strong, citrusy, citral-like, fresh, green, fatty mouthfeel, sweet and floral |
| 3-Hexyl-2-formylthiophene (Structure C5) | Citrusy, citral-like, enhanced mouthfeel and floral |
| 3-Heptyl-2-formylthiophene (Structure C6) | Citrusy, citral-like, fruity, green and slightly salty |
| 3-Octyl-2-formylthiophene (Structure C7) | Citrusy, citral-like, fruity and slightly salty |
| 2-Formylthiophene (Structure 1) | Cherry-like, nutty and sulfurous |
| 3-Methyl-2-formylthiophene (Structure 2) | Fruity, cherry-like, balsamic and savory |
| 3-Ethyl-2-formylthiophene (Structure 3) | Fruity, cherry-like and sulfurous |
| 3-Propyl-2-formylthiophene (Structure 4) | Weak, floral, nutty, waxy, plastic and gasoline-like |
| 3-Nonyl-2-formylthiophene (Structure 5) | Cherry-like, fishy and egg-like |
| 3-Decyl-2-formylthiophene (Structure 6) | Fruity, cherry-like and acetate-like |
| 3-Propenyl-2-formylthiophene (Structure 7) | Fruity, metallic, plastic and vinyl |
| 3-Prop-1-ynyl-2-formylthiophene (Structure 8) | Fruity, plastic and vinyl |
| 3-Cyclopentyl-2-formylthiophene (Structure 9) | Citrusy, citral-like and metallic |
| 3-Cyclopropyl-2-formylthiophene (Structure 10) | Creamy, green and solventy |
| 3-Cyclohexyl-2-formylthiophene (Structure 11) | Cherry-like, plastic, vinyl, solventy , citrusy and weak |
| 4-Butyl-2-formylthiophene (Structure 12) | Citrusy, citral-like, enhanced mouthfeel and cucumber-like |
| 4-Ethyl-2-formylthiophene (Structure 13) | Cherry-like, almond-like, vanilla and balsamic |
| 4-Propyl-2-formylthiophene (Structure 14) | Cherry-like and green |
| 4-Isobutyl-2-formylthiophene (Structure 15) | Green, oily, slightly rancid-like and weak |
| 4-Pentyl-2-formylthiophene (Structure 16) | Green, oily and cucumber-like |
| 4-Hexyl-2-formylthiophene (Structure 17) | Green, floral, woody and cucumber-like |
| 5-Butyl-2-formylthiophene (Structure 18) | Cinnamic, sweet, enhanced mouthfeel, spicy and bitter finish |
| 5-Methyl-2-formylthiophene (Structure 19) | Cherry-like, almond-like, vanilla and balsamic |
| 5-Pentyl-2-formylthiophene (Structure 20) | Citrusy, fruity, green, fatty and metallic |
| 5-Hexyl-2-formylthiophene (Structure 21) | Weak, green, vegetative, fatty and metallic |
| 2-propyl-3-formylthiophene (Structure 22) | Weak, slightly cinnamic, sulfurous, plastic, sweet and chemical |

The evaluation of formylthiophenes in the above examples has surprisingly showed that the 3-substituted formylthiophenes, wherein the substitution is C₄-C₈ straight or branched alkyl or alkenyl group, possess particular desirable and strong citrusy and citral-like notes as well as enhanced mouthfeel, which are not observed in other formylthiophenes. Such advantageous properties of strength and complexity of 3-substituted formylthiophenes are accordingly unexpected and superior.

## Claims

1. A method of improving, enhancing or modifying a composition through the addition of an olfactory acceptable amount of a compound of Formula A: 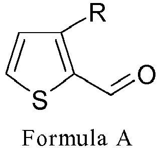
wherein R represents a C₄-C₈ straight or branched alkyl or alkenyl group; and
wherein the composition is a flavor or a fragrance composition.

2. The method of claim 1, wherein the compound is selected from the group consisting of:
3-(3-methyl-but-2-enyl)-2-formylthiophene;
3-(4-methyl-pent-3-enyl)-2-formylthiophene;
3-butyl-2-formylthiophene;
3-isopentyl-2-formylthiophene;
3-isobutyl-2-formylthiophene;
3-pentyl-2-formylthiophene;
3-hexyl-2-formylthiophene;
3-heptyl-2-formylthiophene;
3-octyl-2-formylthiophene; and
a mixture thereof.

3. The method of claim 1, wherein the composition is the flavor composition and the olfactory acceptable amount is greater than 0.1 parts per billion by weight of the flavor composition; or
wherein the composition is the flavor composition and the olfactory acceptable amount is from 1 part per billion to 500 parts per million by weight of the flavor composition.

4. The method of claim 1,wherein the composition is the flavor composition and the olfactory acceptable amount is from 10 parts per billion to 100 parts per million by weight of the flavor composition.

5. The method of claim 1, wherein the composition is the fragrance composition and the olfactory acceptable amount is from 0.005 to 50 weight percent of the fragrance composition.

6. The method of claim 1, wherein the composition is the fragrance composition and the olfactory acceptable amount is from 0.5 to 25 weight percent of the fragrance composition.

7. The method of claim 1, wherein the composition is the fragrance composition and the olfactory acceptable amount is from 1 to 10 weight percent of the fragrance composition.

8. A product comprising an olfactory acceptable amount of a compound of Formula A: 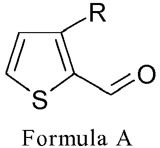
wherein R represents a C₄-C₈ straight or branched alkyl or alkenyl group; and
wherein the product is a cleaning product selected from the group consisting of a detergent, a dishwashing material, a scrubbing composition, a glass cleaner, a metal cleaner, a countertop cleaner, a floor cleaner, a carpet cleaner, a toilet cleaner and a bleach additive.

9. A compound selected from the group consisting of 3-(3-methyl-but-2-enyl)-2-formylthiophene and 3-(4-methyl-pent-3-enyl)-2-formylthiophene.

## Patentansprüche

1. Verfahren zur Verbesserung, Verstärkung oder Modifizierung einer Zusammensetzung durch Zugabe einer olfaktorisch annehmbaren Menge einer Verbindung der Formel A: 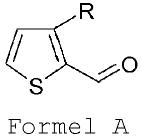
wobei R für eine gerade oder verzweigte C₄-C₈-Alkyl- oder -Alkenylgruppe steht; und
wobei es sich bei der Zusammensetzung um eine Geschmacks- oder Duftstoffzusammensetzung handelt.

2. Verfahren nach Anspruch 1, wobei die Verbindung aus der Gruppe bestehend aus
3-(3-Methylbut-2-enyl)-2-formylthiophen;
3-(4-Methylpent-3-enyl)-2-formylthiophen;
3-Butyl-2-formylthiophen;
3-Isopentyl-2-formylthiophen;
3-Isobutyl-2-formylthiophen;
3-Pentyl-2-formylthiophen;
3-Hexyl-2-formylthiophen;
3-Heptyl-2-formylthiophen;
3-Octyl-2-formylthiophen und
Mischungen davon
ausgewählt ist.

3. Verfahren nach Anspruch 1, wobei es sich bei der Zusammensetzung um die Geschmacksstoffzusammensetzung handelt und die olfaktorisch annehmbare Menge mehr als 0,1 Gewichtsteile pro Milliarde der Geschmacksstoffzusammensetzung beträgt oder
wobei es sich bei der Zusammensetzung um die Geschmacksstoffzusammensetzung handelt und die olfaktorisch annehmbare Menge 1 Gewichtsteil pro Milliarde bis 500 Gewichtsteile pro Million der Geschmacksstoffzusammensetzung beträgt.

4. Verfahren nach Anspruch 1, wobei es sich bei der Zusammensetzung um die Geschmacksstoffzusammensetzung handelt und die olfaktorisch annehmbare Menge 10 Gewichtsteile pro Milliarde bis 100 Gewichtsteile pro Million der Geschmacksstoffzusammensetzung beträgt.

5. Verfahren nach Anspruch 1, wobei es sich bei der Zusammensetzung um die Duftstoffzusammensetzung handelt und die olfaktorisch annehmbare Menge 0,005 bis 50 Gewichtsprozent der Duftstoffzusammensetzung beträgt.

6. Verfahren nach Anspruch 1, wobei es sich bei der Zusammensetzung um die Duftstoffzusammensetzung handelt und die olfaktorisch annehmbare Menge 0,5 bis 25 Gewichtsprozent der Duftstoffzusammensetzung beträgt.

7. Verfahren nach Anspruch 1, wobei es sich bei der Zusammensetzung um die Duftstoffzusammensetzung handelt und die olfaktorisch annehmbare Menge 1 bis 10 Gewichtsprozent der Duftstoffzusammensetzung beträgt.

8. Produkt, umfassend eine olfaktorisch annehmbare Menge einer Verbindung der Formel A: 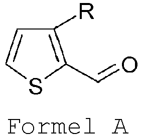
wobei R für eine gerade oder verzweigte C₄-C₈-Alkyl- oder -Alkenylgruppe steht; und
wobei es sich bei dem Produkt um ein Reinigungsprodukt aus der Gruppe bestehend aus einem Reinigungsmittel, einem Geschirrspülmittel, einem Scheuermittel, einem Glasreiniger, einem Metallreiniger, einem Arbeitsplattenreiniger, einem Fußbodenreiniger, einem Teppichreiniger, einem Toilettenreiniger und einem Bleichadditiv handelt.

9. Verbindung, ausgewählt aus der Gruppe bestehend aus 3-(3-Methylbut-2-enyl)-2-formylthiophen und 3-(4-Methylpent-3-enyl)-2-formylthiophen.

## Revendications

1. Procédé d'amélioration, de renforcement ou de modification d'une composition par l'ajout d'une quantité olfactivement acceptable d'un composé de formule A : 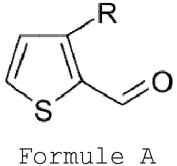
dans lequel R représente un groupe alkyle ou alcényle droit ou ramifié en C₄-C₈ ; et
dans lequel la composition est une composition d'arôme ou de parfum.

2. Procédé selon la revendication 1, dans lequel le composé est choisi dans le groupe constitué par :
le 3-(3-méthylbut-2-ényl)-2-formylthiophène ;
le 3-(4-méthylpent-3-ényl)-2-formylthiophène ;
le 3-butyl-2-formylthiophène ;
le 3-isopentyl-2-formylthiophène ;
le 3-isobutyl-2-formylthiophène ;
le 3-pentyl-2-formylthiophène ;
le 3-hexyl-2-formylthiophène ;
le 3-heptyl-2-formylthiophène ;
le 3-octyl-2-formylthiophène ; et
un mélange de ceux-ci.

3. Procédé selon la revendication 1, dans lequel la composition est la composition d'arôme et la quantité olfactivement acceptable est supérieure à 0,1 partie par milliard en poids de la composition d'arôme ; ou
dans lequel la composition est la composition d'arôme et la quantité olfactivement acceptable est de 1 partie par milliard à 500 parties par million en poids de la composition d'arôme.

4. Procédé selon la revendication 1, dans lequel la composition est la composition d'arôme et la quantité olfactivement acceptable est de 10 parties par milliard à 100 parties par million en poids de la composition d'arôme.

5. Procédé selon la revendication 1, dans lequel la composition est la composition de parfum et la quantité olfactivement acceptable est de 0,005 à 50 pour cent en poids de la composition de parfum.

6. Procédé selon la revendication 1, dans lequel la composition est la composition de parfum et la quantité olfactivement acceptable est de 0,5 à 25 pour cent en poids de la composition de parfum.

7. Procédé selon la revendication 1, dans lequel la composition est la composition de parfum et la quantité olfactivement acceptable est de 1 à 10 pour cent en poids de la composition de parfum.

8. Produit comprenant une quantité olfactivement acceptable d'un composé de formule A : 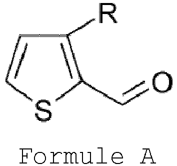
dans lequel R représente un groupe alkyle ou alcényle droit ou ramifié en C₄-C₈ ; et
le produit étant un produit de nettoyage choisi dans le groupe constitué par un détergent, une substance pour laver la vaisselle, une composition de récurage, un nettoyant pour les vitres, un nettoyant pour les métaux, un nettoyant pour les plans de travail, un nettoyant pour les sols, un nettoyant pour les tapis, un nettoyant pour les toilettes et un additif de blanchiment.

9. Composé choisi dans le groupe constitué par le 3-(3-méthylbut-2-ényl)-2-formylthiophène et le 3-(4-méthylpent-3-ényl)-2-formylthiophène.
